(19) Europäisches Patentamt
European Patent Office
Office européen des brevets

(11) Publication number: **0 348 166 B1**

(12) **EUROPEAN PATENT SPECIFICATION**

(45) Date of publication of patent specification: **20.04.94**

(51) Int. Cl.$^5$: **A61K 6/00**, C09J 4/00, C08F 220/00

(21) Application number: **89306251.3**

(22) Date of filing: **20.06.89**

# (54) Adhesive composition containing a thiocarboxylic acid or a derivative thereof.

(30) Priority: **20.06.88 JP 152491/88**
**08.08.88 JP 198623/88**

(43) Date of publication of application:
**27.12.89 Bulletin 89/52**

(45) Publication of the grant of the patent:
**20.04.94 Bulletin 94/16**

(84) Designated Contracting States:
**DE FR GB IT NL**

(56) References cited:
**EP-A- 0 054 942**
**EP-A- 0 277 413**

**PATENT ABSTRACTS OF JAPAN vol. 10, no. 103 (C-340)(2160) 18 April 1986**

(73) Proprietor: **KURARAY CO., LTD.**
**1621 Sakazu**
**Kurashiki-City Okayama Prefecture 710(JP)**

(72) Inventor: **Kawashima, Mitsunobu**
**1652-1, Sakazu**
**Kurashiki-city(JP)**
Inventor: **Omura, Ikuo**
**869-45, Tokubo**
**Kurashiki-city(JP)**

(74) Representative: **Thomas, Roger Tamlyn et al**
**D. Young & Co.**
**21 New Fetter Lane**
**London EC4A 1DA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

### BACKGROUND OF THE INVENTION

#### Field of the Invention

This invention relates to adhesive compositions which provides a high strength bond to metals and particularly to noble metals. More particularly, this invention relates to adhesive compositions which provides a high strength bond to various metals used in the dental field.

#### Description of the Prior Art

Adhesives based on monomers such as acrylic compounds and epoxy compounds, can be allowed to cure at atmospheric temperature and pressure in short periods of time, offering good workability, and as such have been in prevalent use for the bonding of metal. However, depending on the use, these adhesives may prove to have serious drawbacks. Thus, if the bonded product is kept exposed to water, the bond strength suffers a sharp decline, so that these adhesives can hardly be used in applications demanding water resistance.

Recently, in the field of dental materials, various attempts have been made to provide adhesives for metals with improved water resistance of bond strength and been rewarded by some degree of success. For example, a dental adhesive composition containing a phosphoric acid ester compound as disclosed in Japanese laid-open Patent Application KOKAI No. 21607/1983 exhibits an excellent water-resistant bond strength to base metals, such as iron, nickel, chromium, cobalt, tin, aluminum, copper, titanium, etc., and base metal alloys containing such elements as major ingredients, and has actually been in use clinically as a dental cement.

However, the adhesive composition according to said prior art is inferior in water resistance of a bond to noble metal alloys containing gold, platinum, palladium or silver, e.g. inlays, crowns and bridges, to that to base metal alloys.

It has been therefore found necessary, for giving an adequate water resistance to the bond, that such noble metal alloy products should be surface-treated by tin plating or oxidation.

It was recently reported in the Japanese Society for Dental Materials and Devices 5, 92-105, 1986 that a coating of N-(4-mercaptophenyl)methacrylamide as a primer on a noble metal markedly improved the water resistance of a bond of MMA-TBB resin to the noble metal.

As a further example of an earlier document, EP-A-277413 which was published on 10th August 1980 and has a priority date of 6th October 1986 (see Art. 54(3) EPC) describes an adhesive comprising a compound having at least one mercapto group joined to a saturated carbon atom and having at least one olefinic double bond. Also an English-language Abstract of JP-A-60231746 describes a rubber composition which can be firmly bonded to brass and which contains an imidazole dithiocarboxylic acid compound.

Further, Japanese laid-open Patent Application KOKAI No. 110523/1976 states that a varnish based on a methacrylic acid ester was improved in its bond strength to a glass or an aluminum plate by addition of 3-(isopropenylthiocarbonylthio) propyltrimethoxysilane to the varnish.

As mentioned above, when a noble metal or its alloy is bonded to a tooth or a dental material with the adhesive disclosed in Japanese laid-open Patent Application KOKAI No. 21607/1983, the metal surface must be previously treated by tin plating or oxidation and such a procedure requires skill and is time-consuming. This trouble can be obviated by using, instead, the above-mentioned N-(4-mercaptophenyl)-methacrylamide-containing adhesive, but there still remains the problem that the water resistance of the bond is not adequate for practical use.

When the adhesive containing 3-(isopropenylthiocarbonylthio)propyltrimethoxysilane is applied to noble metals or noble metal alloys, the resulting bond strength is not sufficient for practical use.

It is, therefore, an aim of this invention to provide an improved primer for use in the surface conditioning of metals, particularly noble metals, prior to adhesion of the metal to a metal or other material. It is another aim of this invention to provide an improved adhesive for metal-to-metal bonding or metal-to- other material bonding. In this specification, both the primer and the adhesive are collectively referred to as "adhesive" or "adhesive composition".

SUMMARY OF THE INVENTION

This invention is directed to an adhesive composition containing a polymerizable thiocarboxylic acid, or a derivative thereof, of the general formula

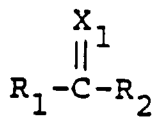 (I)

wherein $X_1$ is an oxygen atom; $R_1$ is an organic group of the general formula

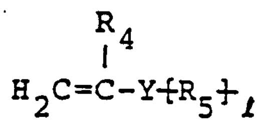

wherein $R_4$ is a hydrogen atom or a methyl group; $R_5$ is an organic group of 1 to 40 carbon atoms; Y is -COO-, -OOC-, -CONH-, -COS-, -SOC-, -S- or

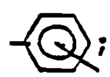 ;

ℓ is 0 or 1, and $R_2$ is a mercapto group; or
wherein $X_1$ is a sulfur atom; $R_1$ is an organic group of the general formula

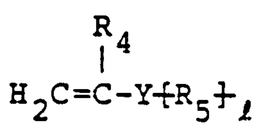

wherein $R_4$ is a hydrogen atom or a methyl group; $R_5$ is an organic group of 1 to 40 carbon atoms; Y is -COO-, -OOC-, -CONH-, -COS-, -SOC-, -S- or

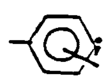 ;

ℓ is 0 or 1; and $R_2$ is a mercapto group or a halogen atom; or
wherein $X_1$ is a sulfur atom; $R_1$ is an organic group of the general formula

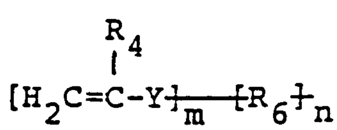

wherein $R_4$ is a hydrogen atom or a methyl group; $R_6$ is an organic group of 1 to 40 carbon atoms; Y is -COO-, -OOC-, -CONH-, -COS-, -SOC-, -S- or

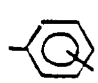 ;

m and n each is 0 or 1 and m + n≧1; $R_2$ is $-OR_3$ or $-SR_3$, where $R_3$ is an organic group of the general formula

$$-[R'_6]_{n'}-[Y'-\overset{\overset{\large R'_4}{|}}{C}=CH_2]_{m'},$$

wherein $R'_4$ is the same as $R_4$; $R'_6$ is the same as $R_6$; Y' is the same as Y; m' and n' each is 0 or 1 and m' + n'≧1; and m + m'≧1.

DETAILED DESCRIPTION OF THE INVENTION

The most outstanding feature of this invention resides in the use of a compound of the above general formula [I] (which will hereinafter be referred to sometimes as the present compound).

The compound of general formula [I] is characterized by the moiety

$$-\overset{\overset{\large X_1}{\|}}{C}-R_2.$$

As specific examples of the

$$-\overset{\overset{\large X_1}{\|}}{C}-R_2$$

group, these may be mentioned

$$-\overset{\overset{\large O}{\|}}{C}-SH,\ -\overset{\overset{\large S}{\|}}{C}-SH,\ -\overset{\overset{\large S}{\|}}{C}-Cl,\ -\overset{\overset{\large S}{\|}}{C}-F,\ -\overset{\overset{\large S}{\|}}{C}-Br,\ -\overset{\overset{\large S}{\|}}{C}-I,\ -\overset{\overset{\large S}{\|}}{C}-O-R_3 \text{ and}$$

$$-\overset{\overset{\large S}{\|}}{C}-S-R_3.$$

In this specification, compounds having any of these groups are called a thiocarboxylic acid or a derivative thereof.

Furthermore, the term 'olefinic double bond' as used in this specification means a carbon-to-carbon double bond which takes part in radical, cationic or anionic addition polymerization to give a high polymer. The term 'organic group' is used in this specification to mean any and all groups that contain 1 to 40 carbon atoms, may contain hydrogen, oxygen, nitrogen, sulfur, phosphorus, and/or halogen in addition to carbon, and meet the following requirements (a) and/or (b).

(a) Hydrocarbon groups in which hydrogen atoms are optionally substituted by -OH, -C≡N, $-NH_2$, $-SO_3H$, halogen, -COOH, -CSSH, -COSH, -COZ, or -CSZ (Z is halogen) and the carbon skeletons may be straight-chain, branched, alicyclic or aromatic or mixtures thereof.

(b) Groups corresponding to at least one of the above-mentioned hydrocarbon groups linked to at least one of the characteristic groups mentioned below, including groups which may be written as -BA, -ABA, wherein A is a hydrocarbon group and B is a characteristic group or groups. The characteristic group B may be exemplified by -O-, -N<,

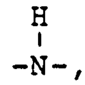

-S-,

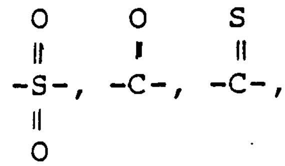

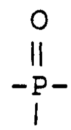 and 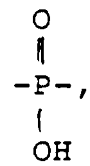

or composites of such characteristic groups, such as

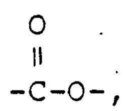 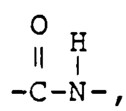 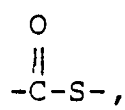

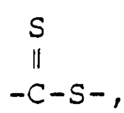 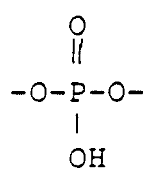

and so on.

It should be understood that where the compound of formula [I] has two or more groups which may be represented by

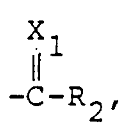

one of such groups is the group

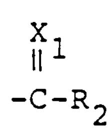

in formula [I], with the other occurring in the organic group $R_1$.

It should also be understood that while any carbothioic acid may exist in the following tautomeric forms, all the carbothioic acids are shown in the thiol form in this specification in accordance with the practice followed by Y. Hirabayashi, T. Mazume, Bull. Chem. Soc. Jpn., 38, 171 (1965), W. W. Crouch, J. Am. Chem. Soc., 74, 2926 (1952), and R. Mecke, H. Spiesecke, Chem. Ber., 89, 1110 (1956).

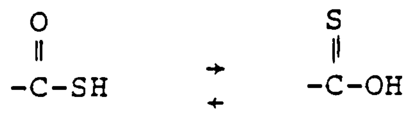

Thiol form Thione form

The first group of compounds of general formula [I] comprises the following carbothioic acids.

Thus, in general formula [I], $X_1$ is an oxygen atom, $R_1$ is an organic group of the general formula

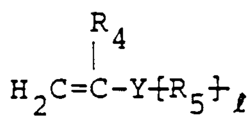

wherein $R_4$ is a hydrogen atom or a methyl group; $R_5$ is an organic group of 1 to 40 carbon atoms; Y is -COO-, -OOC-, -CONH-, -COS-, -SOC-, -S- or

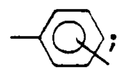

and $\ell$ is 0 or 1; and $R_2$ is a mercapto group.

Therefore, the structural formula of these compounds is as follows.

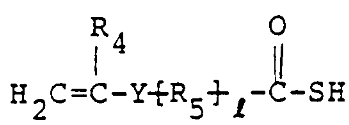 ····· (A) ;

In these carbothioic acids, the moiety containing the olefinic double bond is any of acryloyloxy, methacryloyloxy, vinyloxycarbonyl, isopropenyloxycarbonyl, acryloylamino, methacryloylamino, acryloylthio, methacryloylthio, vinylthiocarbonyl, isopropenylthiocarbonyl, vinylthio, isopropenylthio, vinylphenyl or isopropenylphenyl, and $R_1$ is an organic group containing at least one such olefinic double bond.

The second group of compounds of general formula [I] comprises the following carbodithioic acids or halides thereof. In general formula [I], $X_1$ is a sulfur atom; $R_1$ is an organic group of the general formula

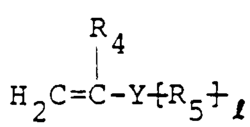

wherein $R_4$ is a hydrogen atom or a methyl group; $R_5$ is an organic group of 1 to 40 carbon atoms; Y is -COO-, -OOC-, -CONH-, -COS-, -SOC-, -S- or

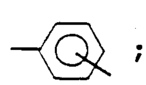

and $\ell$ is 0 or 1; and $R_2$ is a mercapto group or a halogen atom.

Therefore, the structural formula of these compounds is as follows.

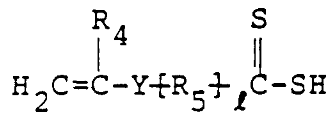 ..... (B), or

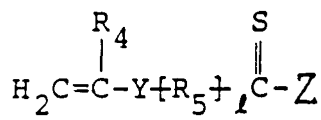 ..... (C) .

In these carbodithioic acids and halides thereof, $R_1$ and Z are the same as that defined for the above-mentioned first group of compounds.

The third group of compounds of general formula [I] comprises the following thiocarboxylic acid derivatives.

In the general formula [I], $X_1$ is a sulfur atom; $R_1$ is an organic group of the general formula

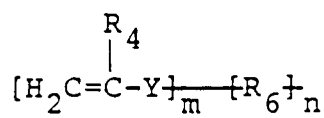

wherein $R_4$ is a hydrogen atom or a methyl group; $R_6$ is an organic group of 1 to 40 carbon atoms; Y is -COO-, -OOC-, -CONH-, -COS-, -SOC-, -S- or

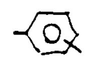 ;

m and n each is 0 or 1 and $m+n \geqq 1$; $R_2$ is $-OR_3$ or $-SR_3$; and $R_3$ is an organic group of the general formula

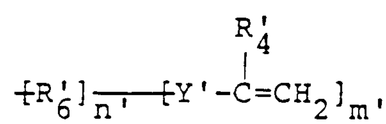

wherein $R'_4$ is the same as $R_4$; $R'_6$ is the same as $R_6$; Y′ is the same as Y; and m′ and n′ each is 0 or 1 and $n'+m' \geqq 1$; $m+m' \geqq 1$.

Therefore, the structural formula of these compounds is as follows.

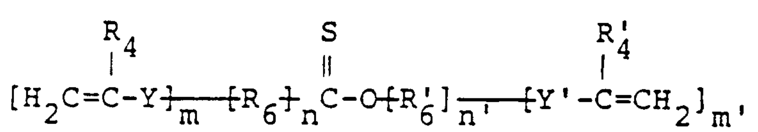 ••• (D), or

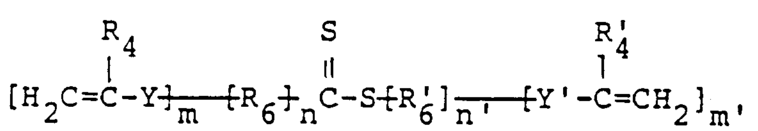 ••• (E).

In these thiocarboxylic acid derivatives, the moiety containing the olefinic double bond is any of acryloyloxy, methacryloyloxy, vinyloxycarbonyl, isopropenyloxycarbonyl, acryloylamino, methacryloylamino, acryloylthio, methacryloylthio, vinylthiocarbonyl, isopropenylthiocarbonyl, vinylthio, isopropenylthio, vinylphenyl or isopropenylphenyl, and at least one of $R_1$ and $R_3$ is an organic group having the above-mentioned olefinic double bond. The thiocarboxylic acid derivatives which can be used in accordance with this invention include the corresponding esters, acid anhydrides and those containing disulfide bonds as

mentioned below in the following list of exemplary compounds.

The following is a list of compounds which can be used in the practice of this invention.

Examples of carbothioic acids

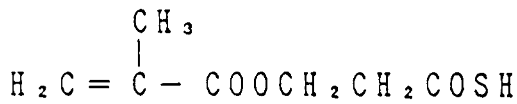

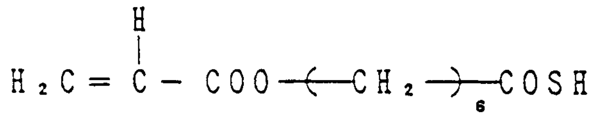

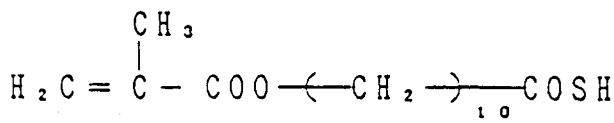

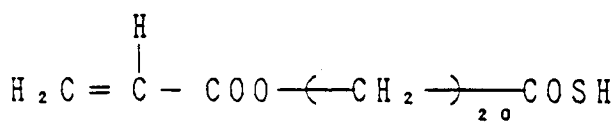

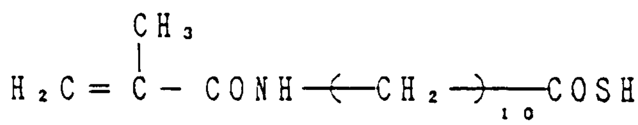

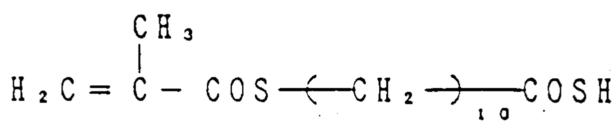

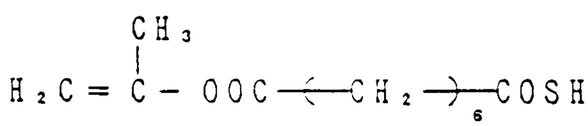

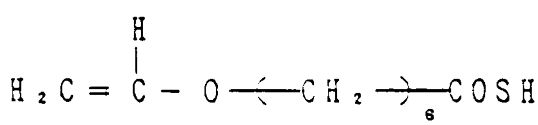

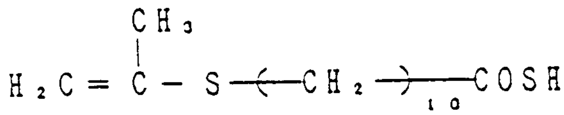

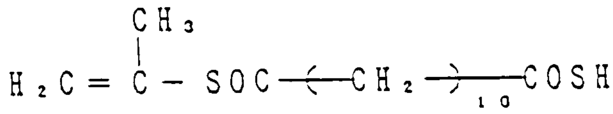

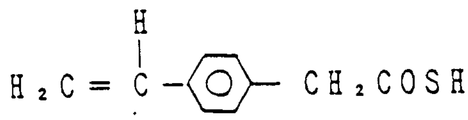

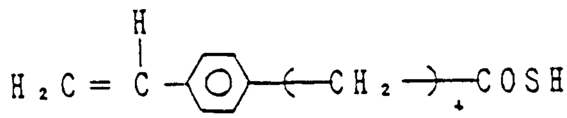

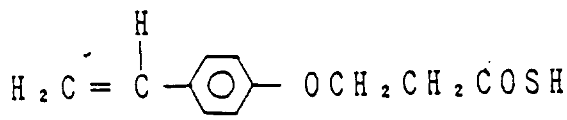

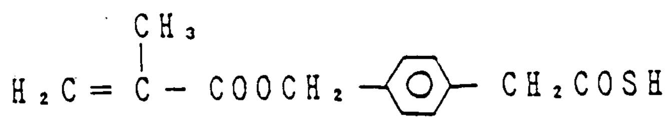

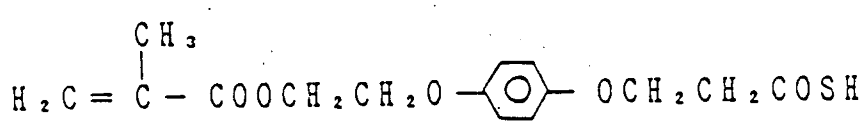

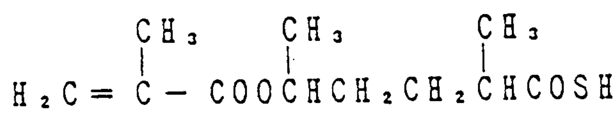

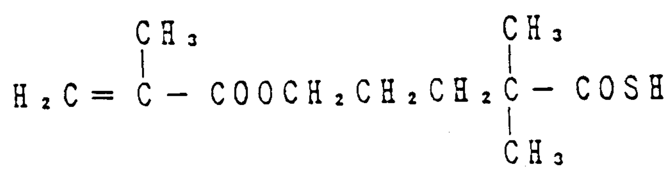

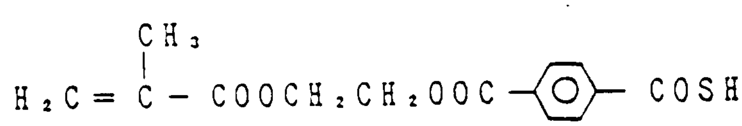

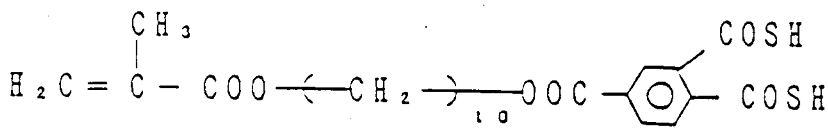

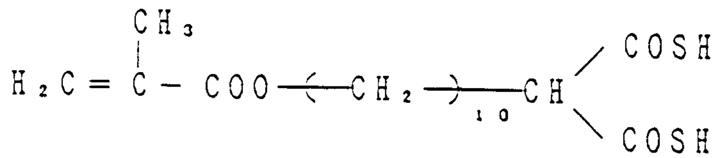

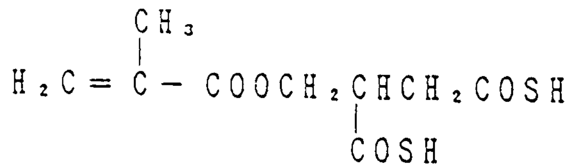

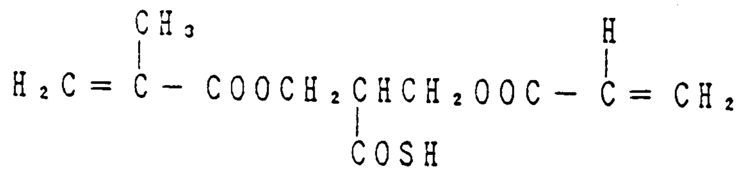

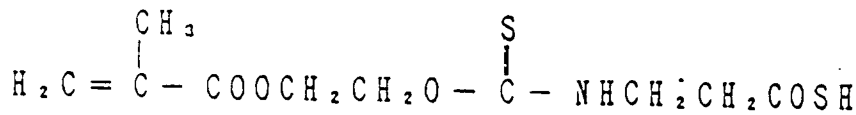

Examples of carbodithioic acids and halides thereof

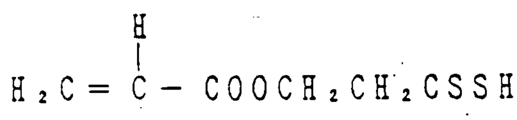

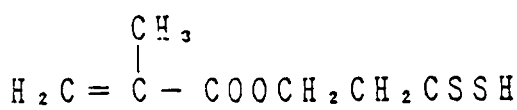

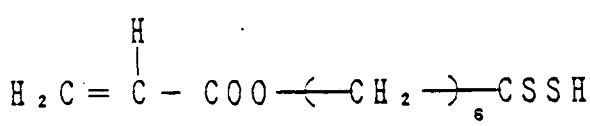

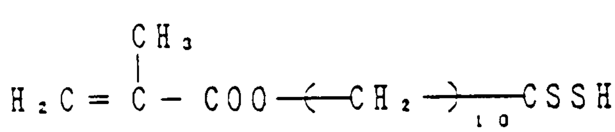

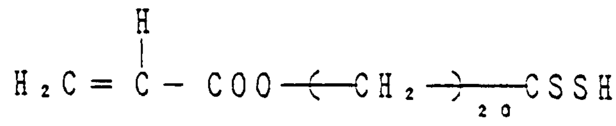

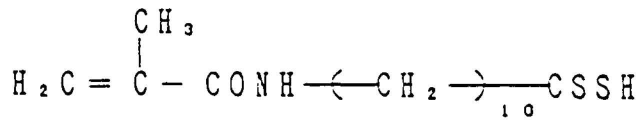

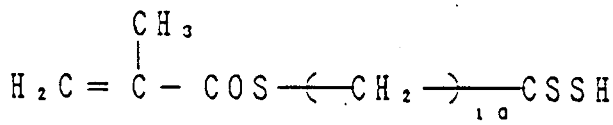

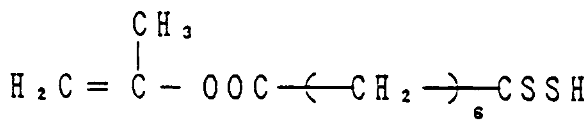

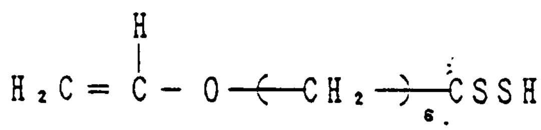

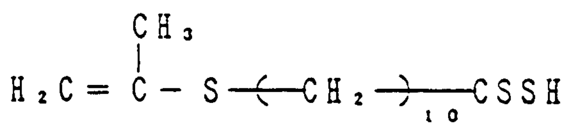

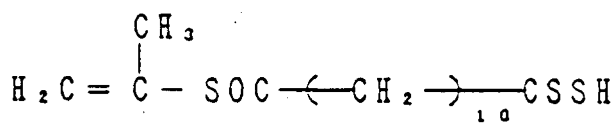

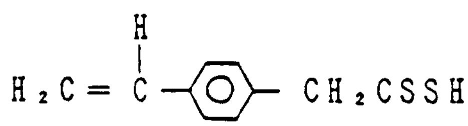

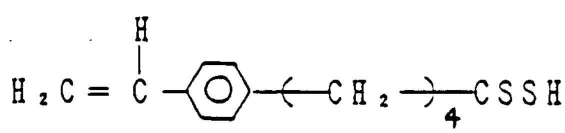

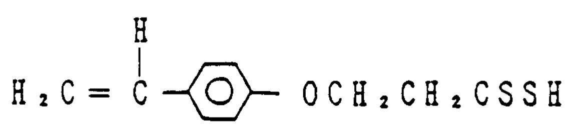

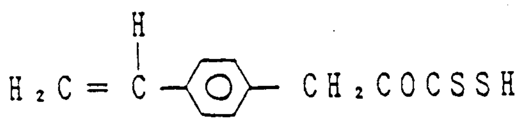

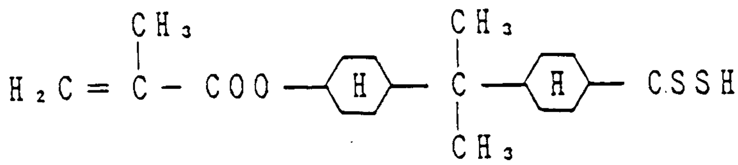

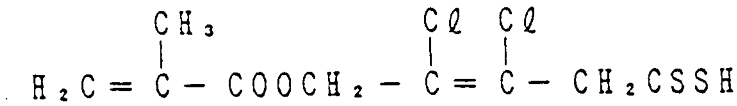

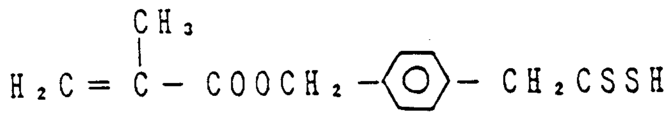

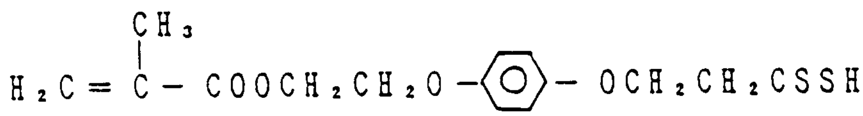

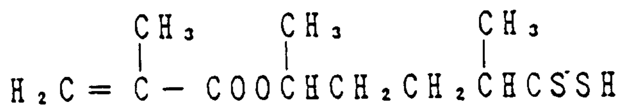

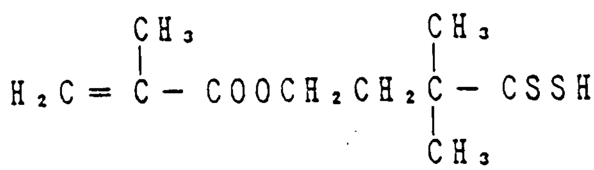

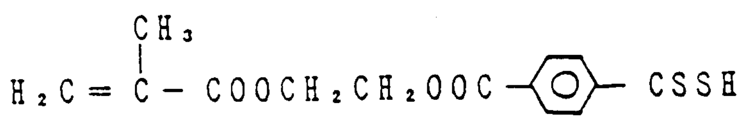

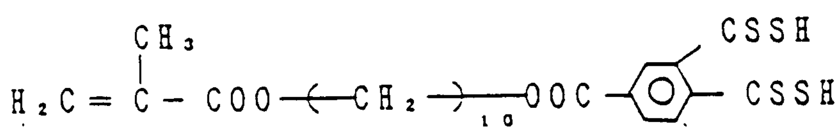

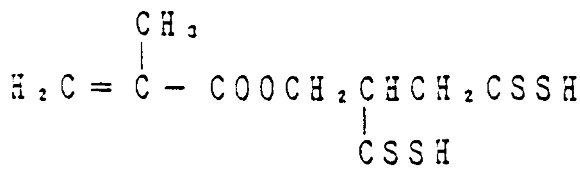

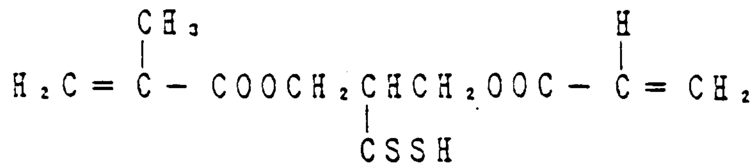

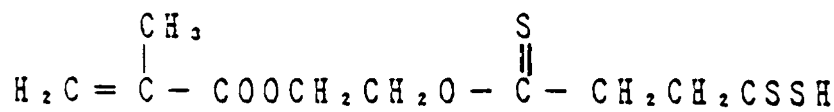

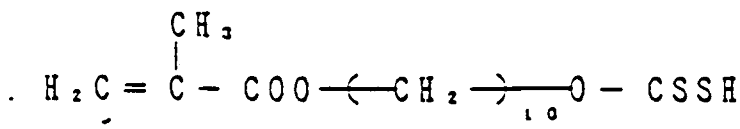

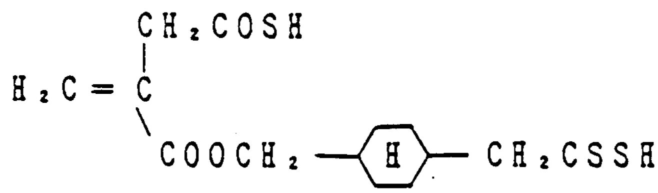

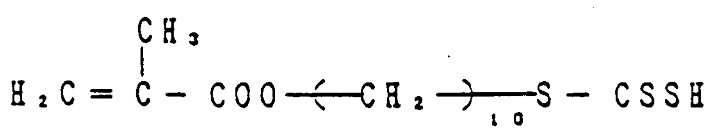

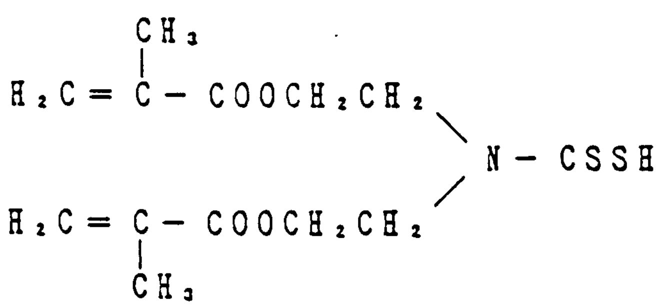

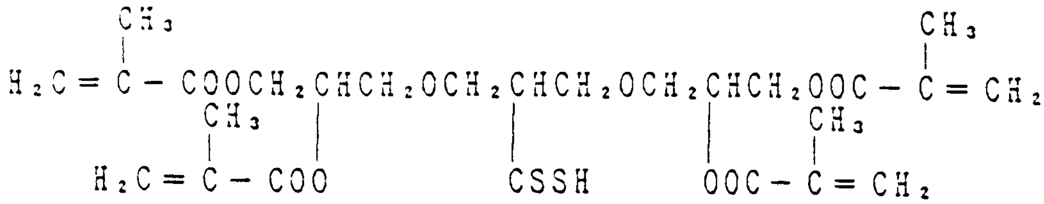

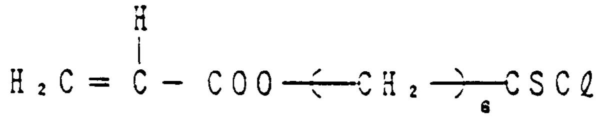

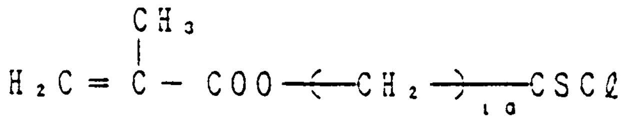

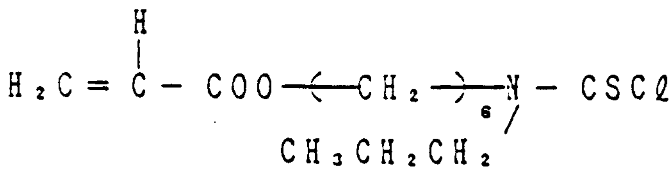

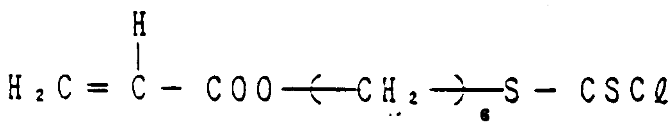

Examples of thiocarboxylic acid derivatives

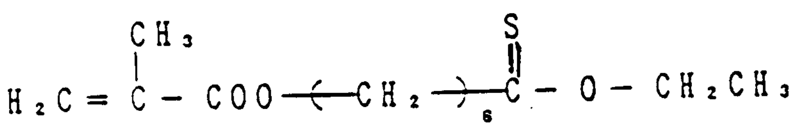

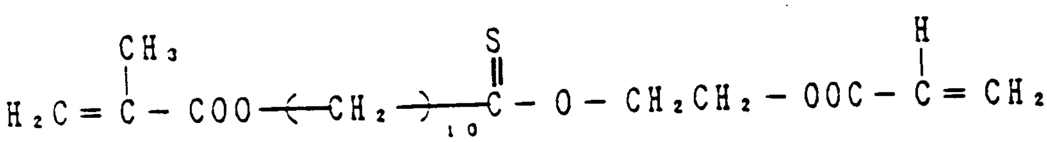

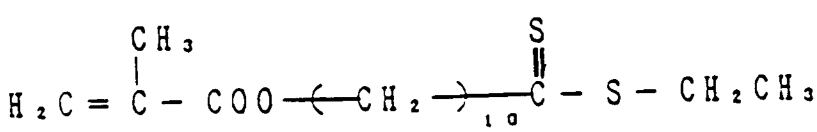

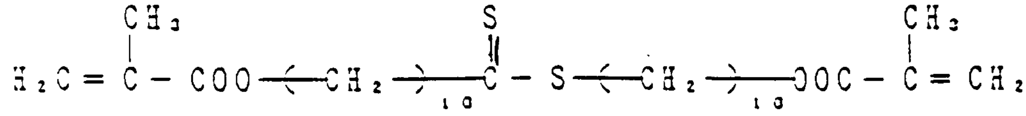

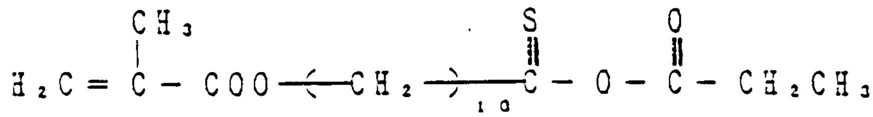

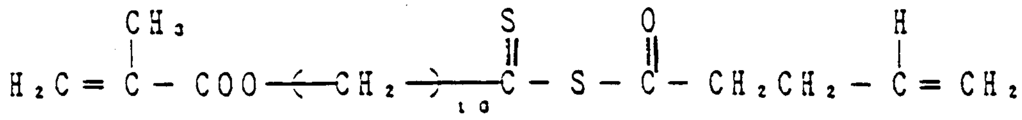

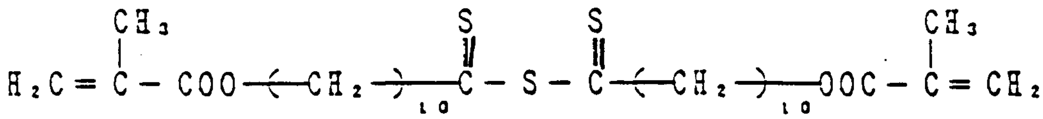

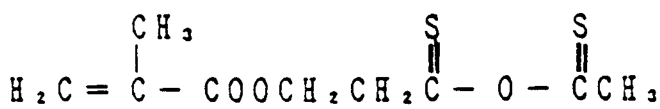

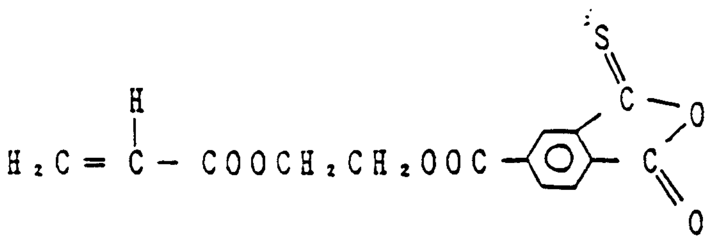

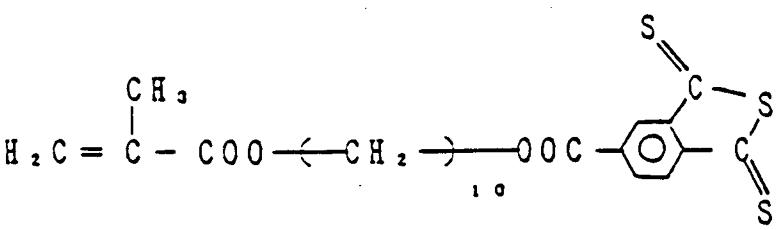

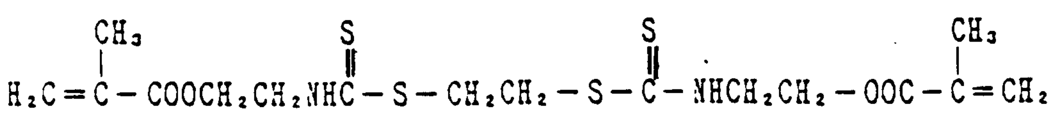

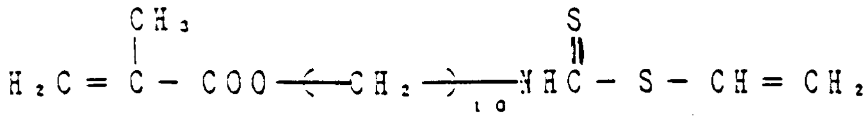

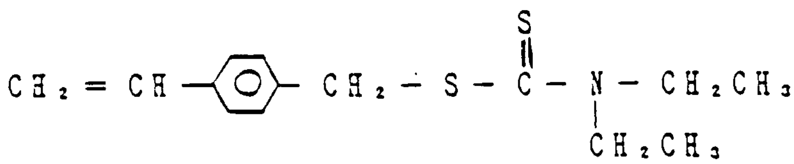

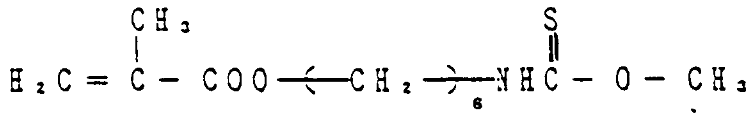

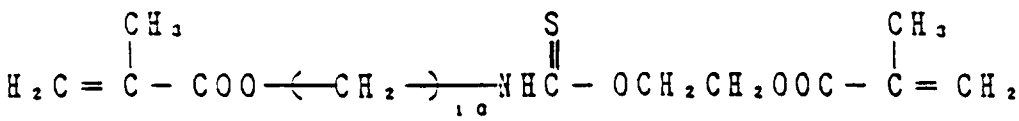

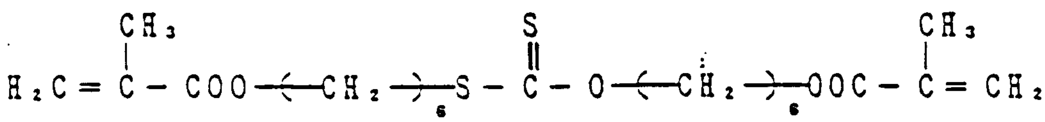

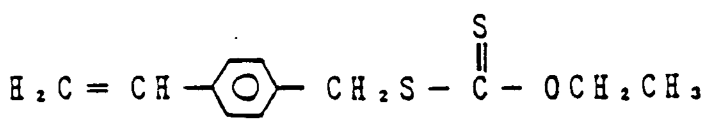

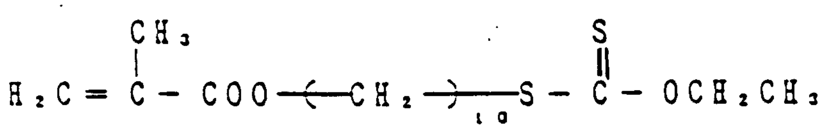

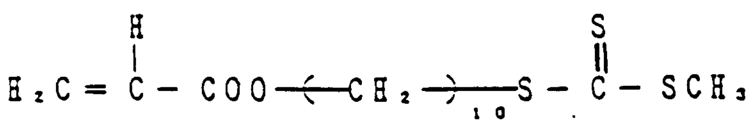

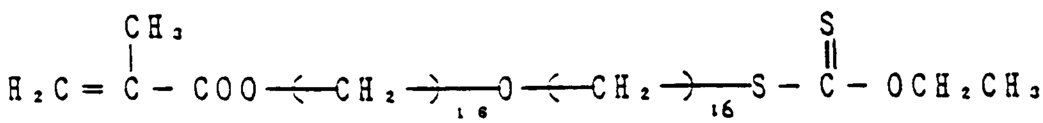

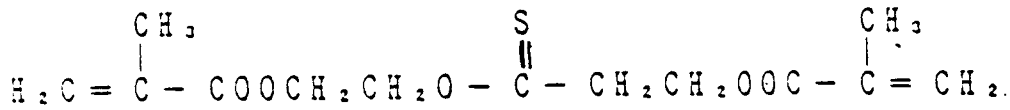

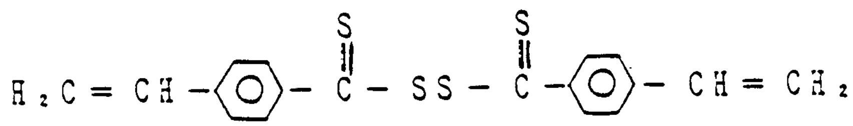

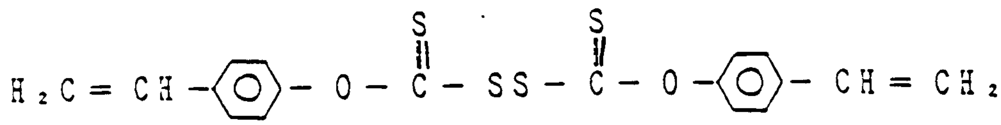

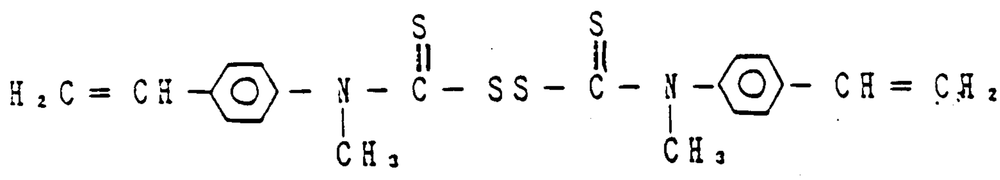

The foregoing compounds to be used as adhesive components in this invention can be synthesized by the processes described, for example, in New Experimental Chemistry Vol. 14 (Maruzen, 1977-1978), The Chemistry of Carboxylic Acids and Esters (John Wiley & Sons, 1969), The Chemistry of Cyanates and Their Thio Derivatives (John Wiley & Sons, 1977), and Comprehensive Organic Chemistry Vol. 3 (Pergamon Press, 1979).

The adhesive composition of this invention is prepared by the following procedure (1) or (2), using the compound or compounds described hereinbefore. As the solvent, those which are either copolymerizable or non-copolymerizable with said polymerizable compounds can be employed.

(1) The present compound is dissolved in a solvent which is non-copolymerizable with it to prepare an adhesive composition. As examples of such solvent, there may be mentioned volatile organic solvents having boiling points not higher than 250 ° C, such as methanol, ethanol, 2-ethylbutanol, acetone, methyl ethyl ketone, diethyl ketone, ethyl ether, n-butyl ether, 1,4-dioxane, tetrahydrofuran, ethyl acetate, toluene, xylene, p-cymene, hexane, octane, methylene chloride, 1,2-dichloroethane, 1,1,2,2-tetrachloroethane, etc., water and mixtures thereof. The concentration of the compound may range from 0.0001 to 99 percent by weight and preferably 0.001 to 50 percent by weight based on the total weight of the compound and solvent. If necessary, a polymerization initiator and/or other copolymerizable monomer can be added.

(2) The present compound is dissolved in a copolymerizable monomer which is described hereinafter. The concentration of the present compound based on the total weight of the compound and comonomer may range from 0.005 to 99 percent by weight, and if necessary, a filler and/or a polymerization initiator is added. The adhesive composition can also be obtained by adding the present compound to an ordinary adhesive agent which consists of a monomer and an initiator and may contain a filler. In such cases, the concentration of the present compound may be in the range of 0.005 to 99 percent by weight based on the total weight of the present compound and comonomer. Moreover, the above-mentioned two types of solvents may be used in combination.

The adhesive composition of this invention can be used not only as an adhesive in the usual manner but also as a primer for other adhesives. For use as a primer, the adhesive composition of this invention is, for example, applied to the surface of a metal and then the ordinary adhesive or composite resin is applied for bonding the metal to the other material.

The fact that the present compound improves the bond strength even when used in a trace amount, is probably attributable to monomolecular adsorption of the compound on the metal surface. This effect is not adversely affected by solvent washing of the primer-coated surface.

The adhesive composition of this invention is preferably used as a primer, and as mentioned above, the adherend surface coated with this primer accepts a conventional adhesive, preferably an acrylic monomer-containing adhesive, to provide a firm bond.

The adhesive of the present invention shows excellent water resistance and when the bonded specimen is kept in water at room temperature, no prominent decrease occurs in bond strength over several months.

The copolymerizable monomer to be used as the solvent for the present compound is preferably a (meth)acrylic acid ester [the term '(meth)acrylic acid ester' means whichever or both of methacrylate and acrylate], such as methyl methacrylate, 2-hydroxyethyl methacrylate, ethylene glycol dimethacrylate, triethylene glycol dimethacrylate, neopentyl glycol dimethacrylate, 2,2-bis[4-(3-methacryloyloxy-2-hydroxypropoxy)phenyl]propane (Bis-GMA), 2,2-bis(methacryloyloxyethoxyphenyl)propane, trimethylolethane triacrylate, pentaerythritol tetraacrylate, 4-(2-methacryloyloxyethyl) trimellitate, 4-methacryloyloxyethyl trimellitate anhydride, bis(2-methacryloyloxyethyl), hydrogen phosphate, 2-methacryloyloxyethyl phenyl hydrogen phosphate, 6-methacryloyloxyhexyl dihydrogen phosphate, 10-methacryloyloxydecyl dihydrogen phosphate, tris(2-methacryloyloxyethyl) phosphate, 2-(dimethylamino)ethyl (meth)acrylate and so on.

As mentioned hereinbefore, a polymerization initiator is usually incorporated in the adhesive composition of this invention. As examples of such polymerization initiator, there may be mentioned benzoyl peroxide-aromatic tertiary amine systems, peroxides such as cumene hydroperoxide, tributylborane, and aromatic sulfinic acid or its salt -aromatic secondary or tertiary amine-acyl peroxide systems. In addition, there may be used photopolymerization initiators such as camphorquinone and camphorquinone-tertiary amine, camphorquinone-aldehyde, camphorquinone-mercaptan and other systems.

When the adhesive composition is to be used as a primer and a different adhesive agent is applied on the primer, it is not essential to incorporate such an initiator in the composition of this invention, for the polymerization initiator contained in the other adhesive agent will migrate to induce polymerization.

If necessary, the adhesive composition of this invention may further contain inorganic fillers such as quartz, glass, hydroxyapatite, calcium carbonate, barium sulfate, titanium dioxide, zirconium oxide, etc., and powders of polymers such as polymethyl methacrylate, polystyrene, polyvinyl chloride and so on.

Among adherend metals to which the adhesive comopsition of this invention is applicable are noble metals such as gold, platinum, palladium, silver, ruthenium, rhodium, osmium, iridium, etc. and a broad range of base metals such as iron, nickel, cobalt, copper, zinc, tin, aluminum, titanium, vanadium, chromium, manganese, zirconium, molybdenum, cadmium, indium, antimony and so on. The adhesive of this invention is also applicable to metal oxides such as aluminum oxide, titanium oxide, zirconium oxide, and further to ceramic materials containing such metal oxides.

Owing to its excellent water resistant adhesiveness to metals and particularly to noble metals, the preferred adhesive composition of this invention finds application with great advantage in the dental field. For example, it is very useful as an adhesive for bonding a noble metal casting such as the inlay, crown, bridge or the like to the tooth, an adhesive for preparing a prosthetic appliance simulating the natural tooth by bonding a resin or ceramic veneer layer to the surface of a noble metal frame, or an adhesive for bonding cast parts of noble metal into a single complete assembly.

It should, however, be understood that the adhesive composition of this invention is not only useful in the dental field but finds application in any other industrial area where bonding of metals or metal oxides is practiced.

The following examples are intended to illustrate this invention in further detail.

## Examples

### Production of the carbothioic acid

#### Production Example 1

In 150 ml of 90% ethanol was dissolved 9 g of potassium hydroxide, and the solution was saturated with hydrogen sulfide under ice-cooling. Then, 30 g of 11 - bromoundecanoyl chloride was added dropwise with constant stirring to the mixture. After completion of the dropwise addition, the temperature was allowed to return to room temperature and the reaction mixture was further stirred for 1 hour. After the insoluble matter was filtered off, the solvent was distilled off under reduced pressure and the residue was dissolved in 150 ml of cold water. Then, neutral impurities were extracted off with benzene. The aqueous solution was acidified with 6N-hydrochloric acid and the liberated organic matter was extracted with ether. This ether layer was washed with water and dried over anhydrous sodium sulfate and the ether was distilled off under reduced pressure to recover 18 g of 11-bromoundecanethioic acid. A mixture of 9.5 g of silver methacrylate, 50 ml of ether and 15 g of the above 11-bromoundecanethioic acid was refluxed for 72 hours and after cooling, the insoluble matter was filtered off. The solvent was then distilled off under reduced pressure and the residue was purified by chromatography. 7.2 g of a solid compound were obtained. By

elemental analysis and NMR, the above compound was identified to be 11-methacryloyloxyundecanethioic acid.

Production of the carbodithioic acid

Production Example 2

A three-necked flask fitted with a dropping funnel, stirrer and reflux condenser was charged with 5 g of magnesium and 100 ml of ether. Then, a solution of 45 g of 10-bromo-1-decene in 200 ml of ether was added via the dropping funnel to prepare a Grignard reagent. With cooling on an ice bath, 15 g of carbon disulfide was added dropwise with stirring. After completion of the dropwise addition, the mixture was stirred on an ice bath for 24 hours, and then ice-water was added to the mixture. The aqueous layer was acidified with diluted hydrochloric acid and the liberated organic matter was extracted with ether. This ether layer was washed with water and dried over anhydrous sodium sulfate and the ether was distilled off under reduced pressure to recover 26 g of 10-undecenedithioic acid. In 100 ml of n-hexane were dissolved 22 g of 10-undecenedithioic acid and 0.2 g of benzoyl peroxide and then, 14 g of dry hydrogen bromide gas was bubbled into the solution at 0°C for 2 hours. Thereafter, the temperature of the mixture was allowed to return to room temperature and the mixture was allowed to stand for 12 hours. The n-hexane was then distilled off to recover 18 g of 11-bromoundecanedithioic acid. A mixture of 9.5 g of silver methacrylate, which was prepared from methacrylic acid, sodium hydroxide and silver nitrate, 60 ml of ether and 15 g of 11-bromoundecanedithioic acid thus obtained was refluxed for 72 hours under heating and the insoluble matter was filtered off after cooling. The solvent was then distilled off from the solution under reduced pressure and the residue was purified by chromatography. 9.2 g of a solid compound were obtained. By elemental analysis and NMR, this compound was identified to be 11-methacryloyloxyundecaneditioic acid.

Production of the carbodithioic acid chloride

Production Example 3

A 50 cc round-bottomed flask was charged with 5 g of 11-methacryloyloxyundecanedithioic acid, 6 g of thionyl chloride, 0.1 g of pyridine and a trace amount of cuprous chloride and the mixture was refluxed at 80°C until the evolution of gases subsided. Then, this flask was connected to a vacuum distillation apparatus and the low-boiling fraction was distilled off. The residue was then purified by chromatography to recover 3.2 g of a liquid compound. By elemental analysis and NMR , this liquid compound was identified as 11 methacryloyloxyundecanethioic acid chloride.

Production of the carbodithioic acid ester

Production Example 4

In a solution of 6 g of potassium hydroxide in 50 ml of methanol was dissolved 20 g of 10-undecenedithioic acid, an intermediate compound in Production Example 2, and 22 g of 10-bromo-1-decene was added dropwise with cooling on an ice bath. The mixture was stirred for 6 hours. The insoluble matter was filtered off and the solvent was then distilled off to recover 9-decenyl 10-undecenedithioate. In 30 ml of n-hexane were dissolved 8 g of the above 9-decenyl 10-undecenedithioate and 0.05 g of benzoyl peroxide and, then, 4 g of dry hydrogen bromide gas was bubbled into the solution at 0°C for 1.5 hours. The temperature of the mixture was allowed to return to room temperature and the mixture was then allowed to stand for 12 hours. The n-hexane was distilled off and the residue was purified by column chromatography. 7.5 g of 10-bromodecyl 11-bromoundecenedithioate were obtained.

A mixture of 4 g of silver methacrylate, 20 ml of ether and 5 g of 10-bromodecyl 11-bromoundeceneditioate thus obtained was refluxed for 96 hours and after cooling, the insoluble matter was filtered off. The solvent was then distilled off from the solution under reduced pressure and the residue was purified by chromatography. 2.8 g of a solid compound were thus obtained. By elemental analysis and NMR, the above compound was identified to be 10-methacryloyloxydecyl 11-methacryloyloxyundecanedithioate.

Adhesives

Examples 1 to 23 and Comparative Examples 1 to 3.

Using the 23 different compounds listed in Table 1 and the conventional compounds mentioned hereinbefore, viz. N-(4-mercaptophenyl)methacrylamide and 3-(isopropenylthiocarbonylthio)-propyltrimethoxysilane, the adhesive effects on noble metals were evaluated.

Each of the above compounds was dissolved in toluene at a concentration of 1 weight percent to give a primer. Then, a pure gold plate (10 × 10 × 1 mm) polished with a silicon carbide abrasive paper (1000 grit) (reinforced with a 4 mm thick stainless steel plate on the reverse side), a dental gold-silver-palladium alloy [Castwell®, GC Dental Industrial Corp., Au = 12%, Ag ≧ 45%, Pd = 20%, 10 x 10 x 1 mm, reinforced in the same manner as above], and a dental gold-platinum-palladium alloy [Degudent Universal, Mitsubishi Metal Corp., Au = 77%, Pt = 10%, Pd = 9%, 10 x 10 x 1 mm, reinforced in the same manner as above] were coated with each of the above toluene solutions using a brush. After 1 minute, the coated surface was washed with pure toluene so as to leave a monomolecular layer adsorbed on the metal surface. Then, an adhesive tape with an aperture 5 mm in diameter was applied to the above surface to prepare an adherend surface. Also, a round bar of SUS 304, 7 mm in diameter and 25 mm long, was provided and the end face of the bar was sand-blasted with alumina abrasive having a particle diameter of 50 µm. On this end face was set up a paste of dental adhesive [Panavia® , Kuraray Co., Ltd.] which comprised 100 weight parts of methacrylic acid ester, 3 weight parts of sodium sulfinate-benzoyl peroxide-tertiary amine polymerization initiator and 320 weight parts of silanated inorganic filler, and the end face of the bar carrying the paste was pressed against said adherend surface for bonding. After one hour, the testpiece was immersed in water at 37 °C. After 24 hours of immersion, the tensile bond strength was measured using a universal materials testing machine (Instron Ltd.) at a crosshead speed of 2 mm/minute. The mean value of 8 testpieces is shown in Table 1.

| | Adhesive component of primer | Bond strength to pure gold ($Kg/cm^2$) | Bond strength to Castwell ($Kg/cm^2$) | Bond strength to Degudent Universal ($Kg/cm^2$) |
|---|---|---|---|---|
| Example 1 | $CH_3$<br>\|<br>$H_2C=C-COOCH_2CH_2COSH$ | 260 | 271 | 308 |
| Example 2 | $CH_3$<br>\|<br>$H_2C=C-COO-(CH_2)_{10}-COSH$<br>(Compound of Production Example 1) | 342 | 353 | 380 |
| Example 3 | $CH_3$<br>\|<br>$H_2C=C-COOCH_2CH_2CSSH$ | 302 | 312 | 320 |
| Example 4 | $H$<br>\|<br>$H_2C=C-COO-(CH_2)_6-CSSH$ | 348 | 366 | 379 |
| Example 5 | $CH_3$<br>\|<br>$H_2C=C-COO-(CH_2)_{10}-CSSH$<br>(Compound of Production Example 2) | 360 | 378 | 389 |
| Example 6 | $CH_3$<br>\|<br>$H_2C=C-CONH-(CH_2)_{20}-CSSH$ | 365 | 372 | 391 |
| Example 7 | $H$<br>\|<br>$H_2C=C-COS-(CH_2)_{10}-CSSH$ | 366 | 387 | 383 |
| Example 8 | $H$<br>\|<br>$H_2C=C-OOC-(CH_2)_6-CSSH$ | 320 | 335 | 346 |

| | Adhesive component of primer | Bond strength to pure gold (Kg/cm$^2$) | Bond strength to Castwell (Kg/cm$^2$) | Bond strength to Degudent Universal (Kg/cm$^2$) |
|---|---|---|---|---|
| Example 9 | 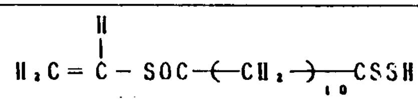 | 352 | 361 | 373 |
| Example 10 | 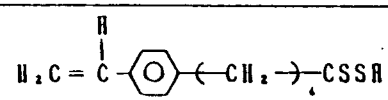 | 351 | 390 | 382 |
| Example 11 | 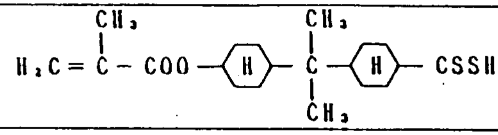 | 346 | 359 | 374 |
| Example 12 | 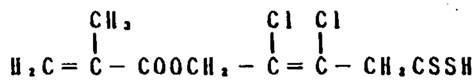 | 328 | 330 | 349 |
| Example 13 | 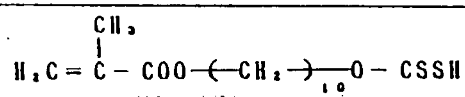 | 358 | 355 | 374 |
| Example 14 | 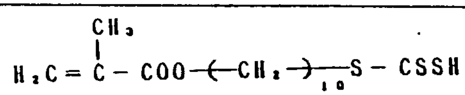 | 361 | 380 | 366 |
| Example 15 | 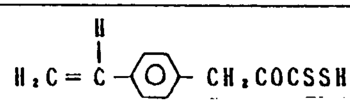 | 342 | 357 | 358 |
| Example 16 | 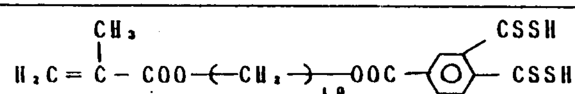 | 368 | 395 | 406 |
| Example 17 | 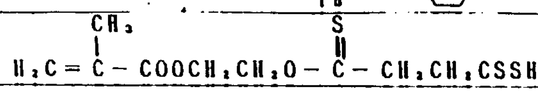 | 356 | 364 | 379 |

| | Adhesive component of primer | Bond strength to pure gold (Kg/cm$^2$) | Bond strength to Castwell (Kg/cm$^2$) | Bond strength to Degudent Universal (Kg/cm$^2$) |
|---|---|---|---|---|
| Example 18 | 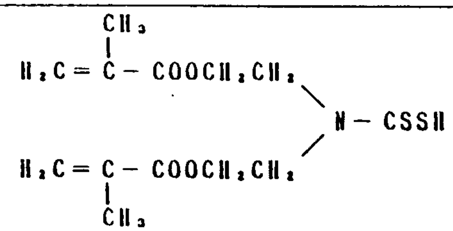 | 348 | 338 | 371 |
| Example 19 | 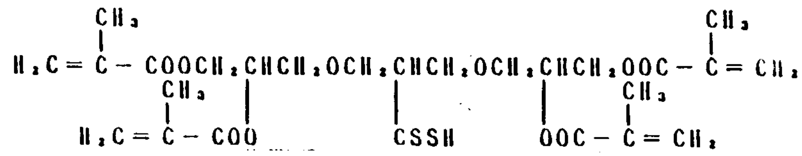 | 314 | 306 | 334 |
| Example 20 | 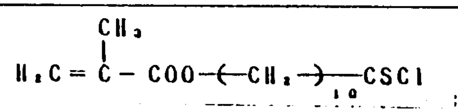 (Compound of Production Example 3) | 337 | 368 | 361 |
| Example 21 | 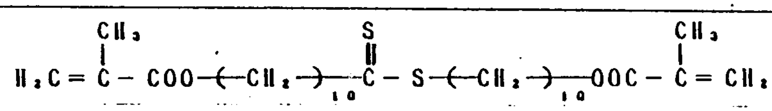 (Compound of Production Example 4) | 295 | 306 | 311 |
| Example 22 | 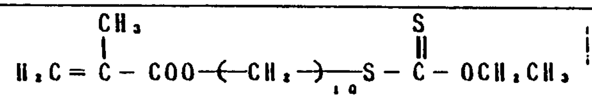 | 278 | 290 | 302 |
| Example 23 | 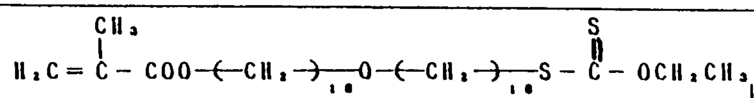 | 282 | 301 | 296 |

| | Adhesive component of primer | Bond strength to pure gold (Kg/cm$^2$) | Bond strength to Castwell (Kg/cm$^2$) | Bond strength to Degudent Universal (Kg/cm$^2$) |
|---|---|---|---|---|
| Example 24 | 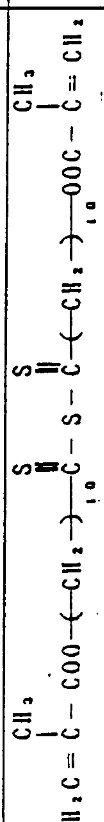 | 319 | 316 | 348 |
| Example 25 | 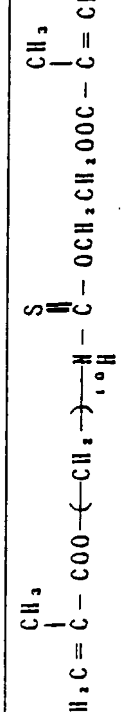 | 270 | 307 | 316 |
| Comparative Example 1 | N-(4-Mercaptophenyl)methacrylamide | 234 | 243 | 173 |
| Comparative Example 2 | 3-(Isopropenylthiocarbonylthio)propyltrimethoxysilane | 202 | 217 | 149 |
| Comparative Example 3 | None | 171 | 151 | 128 |

## Examples 26 and 27 and Comparative Example 4

In the same manner as Example 1, a Castwell® adherend was coated with a 1% toluene solution of the compound used in Example 2 and after a lapse of 1 minute, the coated surface was washed with pure

toluene. Then, a stainless steel bar was adhered using Panavia EX® (Kuraray.Co., Ltd.) to prepare a testpiece (Example 26). Similarly, a testpiece (Example 27) was prepared with the compound used in Example 5. On the other hand, as a control, a testpiece (Comparative Example 4) was prepared by bonding a stainless steel bar directly to a Castwell® adherend with Panavia EX® instead of the adhesive composition of this invention. After 1 hour, for the evaluation of water resistance, these testpieces were immersed in water at 37°C for 24 hours. Each testpiece was transferred into water at 70°C, in which it was allowed to remain for 10 days. Thereafter, the tensile bond strength of each testpiece was measured in the same manner as described in Example 1. As a result, the mean bond strength of Example 26 was 283 kg/$cm^2$ and that of Example 27 was 340 kg/$cm^2$, in contrast to the mean bond strength of 31 kg/$cm^2$ for Comparative Example 4.

Examples 28 and 29 and Comparative Example 5

A two-package adhesive consisting of the following pastes A and B was prepared.

| Paste A | |
|---|---|
| Bis-GMA | 12.5 Weight parts |
| Triethylene glycol dimethacrylate | 12.5 |
| Adhesive component of Example 2 | 0.1 |
| N,N-Diethanol-p-toluidine | 0.5 |
| Silanated quartz powder | 74.5 |

| Paste B | |
|---|---|
| Bis-GMA | 12.5 Weight parts |
| Triethylene glycol dimethacrylate | 12.5 |
| Benzoyl peroxide | 0.5 |
| Silanated quartz powder | 74.5 |

The above pastes A and B were mixed in equal weight parts to prepare an adhesive (Example 28) and by the bonding procedure described in Example 1 (except that a primer was not applied), a dental alloy [Herador H®, Heraeus Edelmetalle GmbH, West Germany; Au = 79%, Pt = 10%, Pd = 8%) adherend polished with silicon carbide abrasive paper (1000 grit) was bonded to a sand-blasted stainless steel bar with the above-prepared adhesive. The adhesion testpiece was immersed in water at 37°C for 24 hours and the tensile bond strength was measured. As a result, an adhesive failure occurred between the gold alloy and the adhesive, and the mean strength (n = 8) was 222 kg/$cm^2$.

A similar experiment was performed with the adhesive component of Example 5 in lieu of that of Example 2. In this case (Example 29), the mean bond strength was 286 kg/$cm^2$.

On the other hand, a paste A′ was prepared by omitting the adhesive component of Example 2 from the composition of paste A, and in the same manner as Examples 28 and 29, a bonding experiment was carried out with the combination of A′ + B (Comparative Example 5). As a result, an adhesive failure occurred between the gold alloy and the adhesive and the mean bond strength was 106 kg/$cm^2$.

Example 30

A powder-liquid mixture adhesive of the following components C and D was prepared.

| Powder C | |
|---|---|
| Silanated silica powder | 100 Weight parts |
| Sodium benzenesulfinate | 0.4 |
| N,N-Diethanol-p-toluidine | 0.5 |

| Liquid D | |
|---|---|
| Bis-GMA | 50 Weight parts |
| 1,6-Hexanediol dimethacrylate | 39 |
| 10-Methacryloyloxydecyl dihydrogen phosphate | 10 |
| Adhesive component of Example 5 | 1 |
| Benzoyl peroxide | 1 |

Using an adhesive paste prepared by blending 1 g of liquid D with 3 g of powder C, a sandblasted stainless steel bar was bonded to a dental alloy [Herador H] adherend polished with silicon carbide abrasive paper (1000 grit) by the same procedure as Example 1 (except, however, that a primer was not applied). The adhesion testpiece was immersed in water at 37°C for 24 hours and the tensile bond strength was measured. As a result, a cohesive failure of the adhesive occurred and the mean bond strength (n = 8) was 328 $kg/cm^2$

Example 31

A disk (4 mm in diameter, 3 mm thick) was cast from Degudent Universal®, a dental alloy, and the surface of the disk was polished with silicon carbide abrasive paper (1000 grit) to prepare an adherend (A). Meanwhile, the labial surface enamel of a human maxillary central incisor was polished with the same abrasive paper as above to prepare a flat surface and this surface was further subjected to acid etching with a 40% aqueous solution of phosphoric acid, rinsed and dried to provide an adherend (B).

The adherend (A) was coated with the primer of Example 2 and after evaporation of the toluene, it was bonded to the adherend (B) using Panavia EX®, and the specimens were obtained. After immersion of the specimens in water at 37°C for a half year, a mean value of tensile bond strength of the 8 specimens was 181 $Kg/cm^2$ and the adhesive failure occured at the interface between enamel and Panavia EX.

**Claims**

1. An adhesive composition comprising a polymerizable thiocarboxylic acid, or a derivative thereof, of the general formula

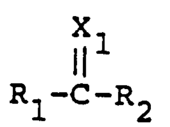

(I)

wherein $X_1$ is an oxygen atom; $R_1$ is an organic group of the general formula

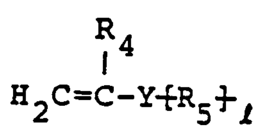

wherein $R_4$ is a hydrogen atom or a methyl group; $R_5$ is an organic group of 1 to 40 carbon atoms; Y is -COO-, -OOC-, -CONH-, -COS-, -SOC-, -S- or

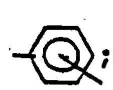;

$\ell$ is 0 or 1, and $R_2$ is a mercapto group; or
wherein $X_1$ is a sulfur atom; $R_1$ is an organic group of the general formula

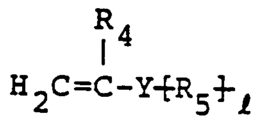

wherein $R_4$ is a hydrogen atom or a methyl group; $R_5$ is an organic group of 1 to 40 carbon atoms; Y is -COO-, -OOC-, -CONH-, -COS-, -SOC-, -S- or

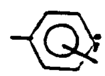

ℓ is 0 or 1; and $R_2$ is a mercapto group or a halogen atom; or
wherein $X_1$ is a sulfur atom; $R_1$ is an organic group of the general formula

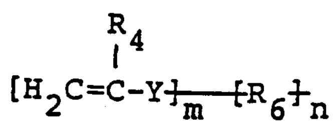

wherein $R_4$ is a hydrogen atom or a methyl group; $R_6$ is an organic group of 1 to 40 carbon atoms; Y is -COO-, -OOC-, -CONH-, -COS-, -SOC-, -S- or

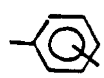 ;

m and n each is 0 or 1 and $m+n \geqq 1$; $R_2$ is $-OR_3$ or $-SR_3$, where $R_3$ is an organic group of the general formula

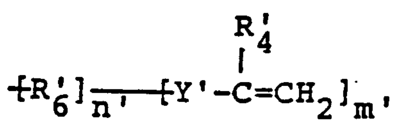

wherein $R_4'$ is the same as $R_4$; $R_6'$ is the same as $R_6$; Y' is the same as Y; m' and n' each is 0 or 1 and $m'+n' \geqq 1$; and $m+m' \geqq 1$.

2. The adhesive composition of claim 1 which comprises the polymerizable thiocarboxylic acid or the derivative thereof and a solvent which is either copolymerizable or non-copolymerizable with said polymerizable compound.

3. Use of the adhesive composition of claim 1 or 2 in the bonding of metals.

4. Use according to claim 3 wherein the adhesive composition is used as a primer for another adhesive composition.

## Patentansprüche

1. Klebstoffzusammensetzung, umfassend eine polymerisierbare Thiocarbonsäure oder ein Derivat davon mit der allgemeinen Formel

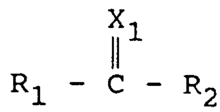

(I)

worin $X_1$ ein Sauerstoffatom bedeutet; $R_1$ eine organische Gruppe der allgemeinen Formel

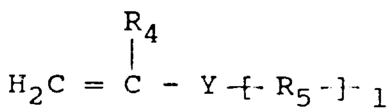

bedeutet, worin $R_4$ ein Wasserstoffatom oder eine Methylgruppe bedeutet; $R_5$ eine organische Gruppe mit 1 bis 40 Kohlenstoffatomen bedeutet; Y die Bedeutung -COO-, -OOC-, -CONH-, -COS-, -SOC-, -S- oder

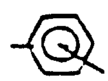

hat; 1 den Wert 0 oder 1 hat und $R_2$ eine Mercaptogruppe bedeutet; oder worin $X_1$ ein Schwefelatom bedeutet; $R_1$ eine organische Gruppe der allgemeinen Formel

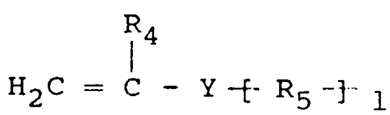

bedeutet, worin $R_4$ ein Wasserstoffatom oder eine Methylgruppe bedeutet; $R_5$ eine organische Gruppe mit 1 bis 40 Kohlenstoffatomen bedeutet; Y die Bedeutung -COO-, -OOC-, -CONH-, -COS-, -SOC-, -S- oder

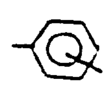

hat; 1 den Wert 0 oder 1 hat und $R_2$ eine Mercaptogruppe oder ein Halogenatom bedeutet; oder worin $X_1$ ein Schwefelatom bedeutet; $R_1$ eine organische Gruppe der allgemeinen Formel

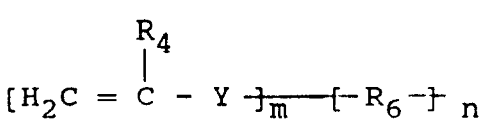

bedeutet, worin $R_4$ ein Wasserstoffatom oder eine Methylgruppe bedeutet; $R_6$ eine organische Gruppe mit 1 bis 40 Kohlenstoffatomen bedeutet; Y die Bedeutung -COO-, -OOC-, -CONH-, -COS-, -SOC-, -S- oder

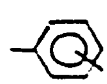

hat; m und n jeweils den Wert 0 oder 1 haben und $m + n \geqq 1$; $R_2$ die Bedeutung $-OR_3$ oder $-SR_3$ hat,

worin $R_3$ eine organische Gruppe der allgemeinen Formel

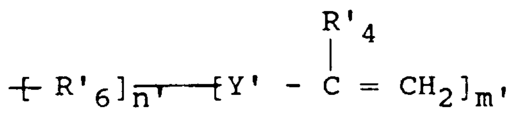

bedeutet, worin $R'_4$ die gleiche Bedeutung wie $R_4$ hat; $R'_6$ die gleiche Bedeutung wie $R_6$ hat; Y' die gleiche Bedeutung wie Y hat; m' und n' jeweils den Wert 0 oder 1 haben und $m' + n' \geqq 1$; und $m + m' \geqq 1$.

2. Klebstoffzusammensetzung nach Anspruch 1, die die polymerisierbare Thiocarbonsäure oder ein Derivat davon und ein Lösungsmittel, das entweder mit der polymerisierbaren Zusammensetzung copolymerisierbar ist oder nicht damit copolymerisierbar ist, umfaßt.

3. Verwendung der Klebstoffzusammensetzung nach Anspruch 1 oder 2 beim Verbinden von Metallen.

4. Verwendung nach Anspruch 3, wobei die Klebstoffzusammensetzung als Primärschicht für eine andere Klebstoffzusammensetzung verwendet wird.

**Revendications**

1. Une composition adhésive comprenant un acide thiocarboxylique polymérisable ou un dérivé de celui-ci, de formule générale

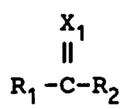

(I)

dans laquelle $X_1$ est un atome d'oxygène ; $R_1$ est un groupe organique de formule générale

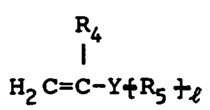

dans laquelle $R_4$ est un atome d'hydrogène ou un groupe méthyle ; $R_5$ est un groupe organique de 1 à 40 atomes de carbone ; Y est -COO-, -OOC-, -CONH-, -COS-, -SOC-, -S- ou

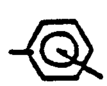 ;

$\ell$ est 0 ou 1 et $R_2$ est un groupe mercapto ; ou
dans laquelle $X_1$ est un atome de soufre ; $R_1$ est un groupe organique de formule générale

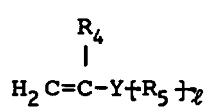

dans laquelle $R_4$ est un atome d'hydrogène ou un groupe méthyle ; $R_5$ est un groupe organique de 1 à 40 atomes de carbone ; Y est -COO-, -OOC-, -CONH-, -COS-, -SOC-, -S- ou

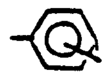 ;

ℓ est 0 ou 1 et $R_2$ est un groupe mercapto ou un atome d'halogène ; ou
dans laquelle $X_1$ est un atome de soufre ; $R_1$ est un groupe organique de formule générale

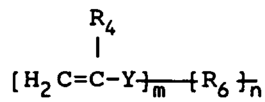

dans laquelle $R_4$ est un atome d'hydrogène ou un groupe méthyle ; $R_6$ est un groupe organique de 1 à 40 atomes de carbone ; Y est -COO-, -OOC-, -CONH-, -COS-, -SOC-, -S- ou

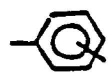 ;

m et n sont chacun 0 ou 1 et $m + n \geqq 1$ ; $R_2$ est $-OR_3$ ou $-SR_3$, où $R_3$ est un groupe organique de formule générale

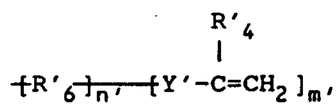

dans laquelle $R'_4$ est identique à $R_4$ ; $R'_6$ est identique à $R_6$ ; Y' est identique à Y ; m' et n' sont chacun 0 ou 1 et $m' + n' \geqq 1$ ; et $m + m' \geqq 1$.

**2.** La composition adhésive de la revendication 1, qui comprend l'acide thiocarboxylique polymérisable ou son dérivé et un solvant qui est soit copolymérisable, soit non copolymérisable avec ledit composé polymérisable.

**3.** Utilisation de la composition adhésive de la revendication 1 ou 2 dans le collage de métaux.

**4.** Utilisation selon la revendication 3, dans laquelle la composition adhésive est utilisée comme primaire pour une autre composition adhésive.